# EUROPEAN PATENT SPECIFICATION

(11) **EP 2 142 166 B1**
(45) Date of publication and mention of the grant of the patent: **11.05.2016**
(21) Application number: 08732514.8
(22) Date of filing: 20.03.2008
(51) Int. Cl.: A61K 8/73, C08L 101/04

(54) **FILMS COMPRISING A PLURALITY OF POLYMERS**
FILME MIT EINER VIELZAHL VON POLYMEREN
FILMS COMPORTANT UNE PLURALITÉ DE POLYMÈRES

(30) Priority: 18.04.2007 US 737050
(43) Date of publication of application: 13.01.2010
(73) Proprietor: Colgate-Palmolive Company, New York, NY 10022 (US)
(72) Inventor: BOYD, Thomas, J., Metuchen, NJ 08840 (US)
(74) Representative: Jenkins, Peter David
(86) International application number: PCT/US2008/057571
(87) International publication number: WO 2008/130764

(56) References cited:
- EP-A- 0 409 254
- EP-A- 1 153 594
- WO-A-01/34121
- WO-A-98/18431
- WO-A-2004/060335
- BE-A- 637 363
- US-A- 4 136 162
- US-A1- 2002 110 536
- US-A1- 2004 086 468
- US-A1- 2006 018 845
- US-A1- 2006 292 088

## Description

### BACKGROUND OF THE INVENTION

The present invention is related to films comprising polymers, compositions comprising such films, and methods for improving the delivery of an active agent to a surface.

Compositions such as oral care compositions, personal care compositions and home care compositions are used for a wide variety of purposes, including the enhancement of personal health, hygiene and appearance, preventing or treating a variety of diseases and other conditions in mammals, and delivery of agents to household surfaces for cleaning, disinfecting, imparting pleasant odors and other benefits. Such compositions may contain films that have functional or active materials contained therein, and that may be stored in a carrier or vehicle of the product. Upon use, the films may degrade by chemical or physical disruption, thereby releasing the active or functional material into the surrounding environment. In this manner, the films provide an opportunity for localized release of a high concentration of active materials near a target surface.

However, there is an ongoing need for improved films having beneficial properties such as increased stability and optimal adhesion to the target surface, as well as methods for improving the stability of oral, personal and home care compositions comprising films to deliver active agents.

US-A-2004/086468 discloses a delivery system for a tooth whitener. EP-A-0409254 discloses a rapid-releasing oral particle pharmaceutical preparation with unpleasant taste masked.

### SUMMARY OF THE INVENTION

The present invention provides a film according to claim 1, comprising:
(a) 5 to 50% of a cellulosic composition chosen from hydroxypropylmethylcellulose or hydroxypropylcellulose based on dry weight; and
(b) 5 to 50% of methyl cellulose based on dry weight;
wherein the breaking strength of the film is greater than 750 psi (5,171 kPa). Preferred features are defined in the dependent claims. The present invention also provides a composition according to claim 5 comprising a film of the present invention in a carrier. Preferred features are defined in the dependent claims.

Also disclosed herein is a film comprising:
(a) a first polymer; and
(b) a second polymer having a solubility temperature lower than that of the first polymer;
wherein the breaking strength of the film is greater than about 750 psi (5,171 kPa).

Also disclosed herein are methods of delivering a film comprising a first polymer, a second polymer having a solubility temperature lower than that of the first polymer, and a breaking strength of greater than about 750 psi (5,171 kPa) to a surface, comprising administering a composition comprising the film to the surface. The breaking strength of the film may be greater than about 1,000 psi (6,894 kPa), 1,100 psi (7,584 kPa), greater than about 1,250 psi (8,618 kPa) or greater than about 1,500 psi (10,342 kPa). The breaking strength of the film may be about 750 psi (5,171 kPa) to about 5,000 psi (34,470 kPa) or about 750 psi (5,171 kPa) to about 2,900 psi (19,995 kPa).

Also disclosed herein is a method for administering an active material comprising applying a film comprising a first polymer and a second polymer having a lower solubility temperature than the first polymer to a human or animal subject, wherein the film comprises the active material, and wherein the ratio of the first polymer to the second polymer is about 1:3 to about 3:1.

Such methods may further comprise disrupting the film after its application to the desired surface. The compositions may comprise a film comprising one or more functional or active materials that are released at different times or concurrently in a controlled release manner, such as by sustained or delayed release.

Also disclosed herein are methods of improving the stability of an oral care formulation, comprising the steps of forming a film by combining a first polymer and a second polymer, wherein the second polymer has a solubility temperature lower than that of the first polymer.

### DETAILED DESCRIPTION OF THE INVENTION

As used herein, ranges are a shorthand for describing each and every value within a range, including endpoints. Where there is a conflict between a definition in the present disclosure and that of a cited reference, the present disclosure controls.

The present invention provides compositions comprising films having desirable physical properties. For example, it has been found that certain oral care compositions can be enhanced by the addition of films that have impregnated therein active ingredients. *See, e.g.,* U.S. Patent No. 6,669,929 to Boyd et al. However, there is still an ongoing need for improved stability and efficacy in such compositions, as well as the achievement of sustained release of active agents. Other problems remain with regard to incompatability of various components in a formulation. For example, organic acids have been found to make some oral care compositions unstable by impacting the solubility or precipitation of polymers, thus leading to overly soluble

(*i.e.,* unstable) films. This kind of disadvantage can lead to undesirable instability problems in all realms of consumer products, as organic acids such as citric acid, gluconic acid and other polymeric acids and polyacryclic acids are commonly found oral care, personal care and home care applications.

The compositions and methods of the present invention provide superior ability to release active agents, based on the discovery that providing a film with at least two polymers having different solubility temperatures results in films that are able to provide a desirable ongoing rate of release of active agent. The compositions of the present invention are advantageous in that they exhibit benefits such as enhanced stability and delivery profiles for oral care, personal care and home care compositions comprising films.

Described herein is a film comprising:
(a) a first polymer; and
(b) a polymer having a solubility temperature lower than that of the first polymer;
wherein the breaking strength of the film is greater than about 750 psi (5,171 kPa). As used herein, the term "solubility temperature" refers to the temperature at which a given water soluble polymer in its solid form dissolves in a solvent. In the case of many cellulosic polymers such as methylcellulose, hydroxypropyl methyl cellulose and hydroxypropyl cellulose, the term "gelation temperature" may be used to indicate the temperature above which such polymers are generally not soluble in water.

At least one of the first polymer or second polymer may be a water soluble polymer, water dispersible polymer, or a mixture thereof. Where at least one of the polymers is water soluble, it need not be completely water soluble, so long as a solubility temperature can be measured. At least one of the first polymer or second polymer may be a cellulosic polymer. At least one of the first polymer or second polymer may be hydroxypropylmethylcellulose (HPMC), hydroxypropylcellulose (HPC), or methylcellulose (MC). The first polymer may be hydroxypropylmethylcellulose (HPMC) and the second polymer may be methylcellulose (MC).

At least one of the water soluble polymers can be chosen from polyvinylpyrrolidone, polyacrylic acid, polymethylacrylic acid, carboxymethyl cellulose, polyvinylalkyl ether-maleic acid copolymer (available under the trade name Gantrez), and various naturally derived polymers, such as xanthan gum, locust bean gum, chitosan and hyaluronic acid.

The solubility temperature of the polymers can be determined according to the Solubility Temperature Test, which is performed as follows:

### The Solubility Temperature Test

The solubility temperature is defined for a single polymer, cast into a film and cut into a 1 inch (2.54 cm) diameter circle at or above 40% by weight (see the Example for an illustrative single polymer film formulation). Film compositions of 2 mils (0.051 mm) thickness are prepared with a polymer. The Dissolution Time is measured - that is, the time it takes a film to disperse in an 11 inch by 17 inch (28 cm x 43 cm) pan filled to 1 inch (2.54 cm) deep with quiescent water, such that the piece of polymer film is no larger than about ¼ inch by about ¼ inch (0.64 cm x 0.64 cm). At higher water temperatures, the Dissolution Time will increase for certain polymers (such as cellulose ethers). The Solubility Temperature is the water temperature at which the Dissolution Time of the film is first measured to be 15 minutes or longer.

The first polymer may have a solubility temperature greater than about 100 degrees F (37.8°C), about 100 degrees F (37.8°C) to about 200 degrees F (93.3°C), and about 105 degrees F (40.6°C) to about 170 degrees F (76.7°C). The second polymer may have a solubility temperature of less than about 100 degrees F (37.8°C), about 80 degrees F (26.7°C) to about 140 degrees F (60.0°C), or about 100 degrees F (37.8°C) to about 150 degrees F (65.6°C).

The present invention provides a film comprising: (a) 5 to 50% of a cellulosic composition chosen from hydroxypropylmethylcellulose or hydroxypropylcellulose based on dry weight; and (b) 5 to 50% of methyl cellulose based on dry weight; wherein the breaking strength of the film is greater than 750 psi (5,171 kPa). In certain embodiments, the film comprises: (a) 10 to 40% hydroxypropylmethylcellulose or hydroxypropylcellulose based on dry weight; and (b) 10 to 40% of methyl cellulose based on dry weight. The cellulosic composition of (a) may be hydroxypropylmethylcellulose.

In certain embodiments, the present invention provides compositions comprising a film of the present invention in a carrier.

In certain embodiments, the film is suitable for use in an oral care composition, a personal care composition (e.g., a hair care composition or a skin care composition) or a home care composition. In various embodiments, the film comprises a functional or active material may be chosen from an oral care active, a personal care active or a home care active.

### Film:

The embodiments of the present are directed to films and compositions that comprise a film. As referred to herein, a "film" is a material that may have substantially lamellar structure, or alternatively may have a substantially non-lamellar structure, e.g., a particle.

A "lamellar" structure has, or is capable of having, a size in one or two dimensions (e.g., the x- or y-dimensions) that is substantially greater than the thickness of the structure in a third dimension (e.g., the z-direction). Useful lamellar structures may include those that are substantially planar, layered, or lamelliform. In various embodiments, the lamellar structure may be substantially planar (having a size in both the x- and y- dimensions that is substantially greater than the z- direction) or non-planar. In various embodiments, the film may comprise a substantially continuous surface that can appear as a substantially flat surface, or may be deformed. The film can have any of a number of shapes.

In contrast, in embodiments wherein the film is non-lamellar, it may be a particle, having, for example, a spherical, spheroid, oblong, or otherwise irregularly shaped and/or having uniform thickness, in contrast to lamellar, form.

In the embodiments of the present invention, the film comprises at least a first polymer and a second polymer. Useful polymers include water soluble polymers, water dispersible polymers or mixtures thereof.

In certain embodiments, at least one of the first polymer or second polymer is a water soluble, breakable polymer that dissolves during use such as, for example, during the brushing of teeth, the application of a composition to the skin or hair, or the application of a composition to a household surface such as a hard surface or article of clothing (e.g., scrubbing or wiping). Such dissolution can occur as a result of, for example, shearing and/or exposure to a solvent comprising a high concentration of water (including saliva). In some embodiments, the polymer is insoluble but breakable in water by being dispersible, *i.e.,* the polymer breaks down into small fragments, for example, as a result of shearing. In some embodiments, a film fragment can comprise a mixture of film forming materials. Water soluble or water dispersible polymers that may be useful for the present embodiments include cellulose ethers, methacrylates, polyvinylpyrollidone, and mixtures thereof. In certain embodiments, the first polymer is a cellulose ether, such as one chosen from hydroxyalkyl cellulose polymers such as hydroxypropyl methyl cellulose (HPMC), hydroxypropyl cellulose (HPC), methyl cellulose (MC), carboxymethyl cellulose and mixtures thereof. For example. HPMC has an incipient gelation temperature of about 45 to about 55 degrees C at a 10% weight concentration, and MC has an incipient gelation temperature of about 25 to about 35 degrees C at a 10% weight concentration (Dow Technical Handbook. "Methocel Cellulose Ethers," September 2002).

Other useful polymers include polyvinylpyrrolidone, cross-linked polyvinyl pyrrolidone, polyvinylpyrrolidone-vinyl acetate copolymer, polyvinylalcohol, polyacrylic acid, polyacrylate polymer, polyethylene oxide, polypropylene oxide, co-polymers of ethylene oxide and propylene oxide (*i.e.,* poloxamers), polyethylene glycol, polyvinyl alkyl ether-maleic acid copolymer (such as Gantrez®) and carboxy vinyl polymer; natural gums such as sodium alginate, carrageenan, xantham gum, gum acacia, arabic gum, guar gum, pullulan, agar, chitin, chitosan, pectin, karaya gum, zein, hordein, gliadin, locust bean gum, tragacantha and other polysaccharides; starches such as maltodextrin, amylose, high amylose starch, corn starch, potato starch, rice starch, tapioca starch, pea starch, sweet potato starch, barley starch, wheat starch, waxy corn starch, modified starch (e.g.. hydroxypropylated high amylose starch), dextrin, levan, elsinan and gluten: and proteins such as collagen, whey protein isolate, casein, milk protein, soy protein and gelatin.

In certain embodiments, at least one of the first polymer or second polymer is a hydroxyalkyl cellulose such as hydroxypropyl methyl cellulose, hydroxyethylpropyl cellulose, hydroxybutyl methyl cellulose, hydroxy propyl cellulose or carboxymethyl cellulose. In certain embodiments, the first polymer is a low viscosity HPMC. When HPMC is used as the film forming agent, it may have a viscosity in the range of about 1 to about 1000 mPa·s as determined as a 2% by weight aqueous solution of the HPMC at 20°C using a Ubbelohde tube viscometer. In certain embodiments the HPMC has a viscosity of about 3 to about 500 mPa·s at 20°C. HPMC is available commercially, for example, from the Dow Chemical Company under the trade designation Methocel, including as Methocel E5LV. Methocel E50, and Methocel K100. Methocel E5 LV is a USP grade, low viscosity HPMC having 29.1 % methoxyl groups and 9% hydroxyproxyl group substitution, and is a white or off-white free-flowing dry powder. As a 2 wt.% solution in water as measured with a Ubbelohde tube viscometer, it has a viscosity of 5.1 mPa·s at 20°C. In various embodiments, at least one of the first water soluble polymer or second water soluble polymer is incorporated in the film matrix in amounts ranging about 10 to about 80% by weight, about 20 to about 75% by weight, or about 30 to about 60% by weight.

In various embodiments, the film disintegrates during use of the composition. In some embodiments, the film releases the functional or active material into the carrier. As referred to herein. "disintegrate" refers to physical disruption of the film or fragment material, so as to produce a film or film fragments of reduced size compared to the original film. Such disruption may be through mechanical, chemical means, or a combination thereof. The disintegration can result, for example, from shearing, grinding, scrubbing (as with a brush or other implement), or exposure to elevated temperatures or solvents such as water or saliva during use; or from change in pH or breakdown through enzymes.

The film of the present invention optionally comprises materials that affect the physical or functional characteristics of the film. Such additional substances can be, for example, emulsifiers, plasticizers, fillers, or texture modifiers. Fillers may include inert starch particles and cellulose. Texture modifiers may include cold water swellable, physically modified and pregelatenized starches, to increase the stiffness of polymeric films.

### Functional or Active Material:

The films of the present invention may comprise a functional or active material. Additionally, in the embodiments of the present invention directed to compositions comprising films, the compositions may further comprise a functional or active material. As referred to herein, the terms "functional material" or an "active material" are used interchangeably, and refer to a material having a desired utility in the oral, personal or home care area. In various embodiments, such utilities may be therapeutic, cosmetic, aesthetic, decorative, cleansing, disinfecting, bleaching, descaling, sensory or combinations thereof.

The functional materials may be, for example, adsorbed to the surface of the film, incorporated within the film or comprise one or more coatings on the surface of the film. Certain embodiments may be directed to oral care, personal care or home care compositions comprising one or more of a first film fragment having a first functional material, and one or more of a second film fragment having a second functional material that differs from the first functional material.

In various embodiments, the functional material may be a flavorant. In certain oral care embodiments, a flavorant may be rapidly released as the fragments disintegrate during use of the product, delivering a breath freshening flavor, desired mouthfeel or sweetness into the oral cavity. Useful flavorants may include synthetic flavor oils or a flavoring aromatics, oleo resins and extracts derived from plants, leaves, flowers, fruits and combinations thereof, as well as sweeteners. In certain embodiments, the film comprises flavoring or food additive, such as those described in Chemicals Used in Food Processing, publication 1274 by the National Academy of Sciences, pages 63-258. In various embodiments, the film comprises a flavorant at a level of about 0.01 to about 15%, about 0.1 to about 12%, or about 1 to about 10% by weight of the film.

In various embodiments, the film may comprise a therapeutic active. As referred to herein, a "therapeutic active" is a material useful for the prevention or treatment of a physiological disorder or condition. Such disorders or conditions include those of the oral cavity (including the teeth and gingiva), skin, hair, and eyes. The specific therapeutic active is preferably determined according to the desired utility of the composition. Such actives may include the following:
- antimicrobial agents, such as triclosan, cetyl pyridium chloride, domiphen bromide, quaternary ammonium salts, zinc compounds, sanguinarine, fluorides, alexidine, octonidine, EDTA,
- essential oils such as thymol, methyl salicylate, eucalyptol and menthol, and the like.
- non-steroidal anti-inflammatory drugs, such as aspirin, acetaminophen, ibuprofen, ketoprofen, diflunisal, fenoprofen calcium, naproxen, tolmetin sodium, indomethacin, and the like.
- anti-tussives, such as benzonatate, caramiphen edisylate, menthol, dextromethorphan hydrobromide, chlophedianol hydrochloride, and the like,
- decongestants, such as pseudoephedrine hydrochloride, phenylepherine, phenylpropanolamine, pseudoephedrine sulfate, and the like,
- antihistamines, such as brompheniramine maleate, chlorpheniramine maleate, carbinoxamine maleate, clemastine fumarate, dexchlorpheniramine maleate, diphenhydramine hydrochloride, diphenylpyraline hydrochloride, azatadine meleate, diphenhydramine citrate, doxylamine succinate, promethazine hydrochloride, pyrilamine maleate, tripelennamine citrate, triprolidine hydrochloride, acrivastine, loratadine, brompheniramine, dexbrompheniramine, and the like,
- expectorants, such as guaifenesin, ipecac, potassium iodide, terpin hydrate, and the like.
- anti-diarrheals, such a loperamide, and the like,
- H₂ -antagonists, such as famotidine, ranitidine, and the like,
- proton pump inhibitors, such as omeprazole, lansoprazole, and the like.
- general nonselective CNS depressants, such as aliphatic alcohols, barbiturates and the like,
- general nonselective CNS stimulants such as caffeine, nicotine, strychnine, picrotoxin, pentylenetetrazol and the like,
- drugs that selectively modify CNS function such as phenyhydantoin, phenobarbital, primidone, carbamazepine, ethosuximide, methsuximide, phensuximide, trimethadione, diazepam, benzodiazepines, phenacemide, pheneturide, acetazolamide, sulthiame, bromide, and the like,
antiparkinsonism drugs such as levodopa, amantadine and the like, and
- narcotic-analgesics such as morphine, heroin, hydromorphone, metopon, oxymorphone, levorphanol, codeine, hydrocodone, xycodone, nalorphine, naloxone, naltrexone and the like, analgesic-antipyretics such as salycilates, phenylbutazone, indomethacin, phenacetin and the like, psychopharmacological drugs such as chlorpromazine, methotrimeprazine, haloperidol, clozapine, reserpine, imipramine, tranylcypromine, phenelzine, lithium and the like.

In certain oral care, personal care or home care embodiments of the present invention, useful functional materials may include, for example: flavorants, fragrances, essential oils, emulsifying agents, thickening agents, colorants, cooling agents, sweeteners, binding agents, sulfur precipitating agents, plasticizing agents, pharmaceutical actives, salivary stimulants, stain prevention actives, anti-microbial agents, anticaries agents, anticalculus agents, antiplaque agents, periodontal actives, breath freshening agents, malodor control agents, whitening agents, vitamins, herbs and herbal extracts, amino acids, enzymes or other proteins, steroids, anti-inflammatory agents, abrasives, antiperspirant actives, deodorant actives, conditioning agents, moisturizers, emollients, sunscreens, sunblocks, alcohols, denaturants, anti-dandruff agents, anticholinergics, anesthetics, foaming agents, surfactants, cleansing agents, bleaches, detergents, fabric softening agents, preservatives and combinations thereof. Useful active materials are also described in U.S. Patent 6,596,298 to Leung et al. In various embodiments, the films comprise such active materials at a concentration of about 0.001 to about 50% by weight of film, about 0.01 % to about 40% by weight of the film, about 0.1 to about 30% by weight of the film or about 1% to about 25% by weight of film.

In various embodiments, the present invention provides a dentifrice having suspended or embedded therein flakes of a water hydratable film comprised of a mixture of a water soluble cellulosic polymer and a second polymer having a solubility temperature lower than that of the first polymer. In various embodiments, at least one of the first polymer or second polymer is HPMC. In various embodiments, the first polymer is HPMC and the second polymer is MC.

The films of the present invention may be made in a variety of ways, including methods among those known in the art for making films. In various embodiments, components of a film forming slurry, such as those disclosed in the Examples below, are mixed to form a film forming slurry composition. The slurry is cast on a releasable substrate and dried to form a sheet of film material. In certain embodiments, the substrate material has a surface tension that allows the film slurry to spread substantially uniformly across the substrate surface, thereby avoiding formation of a destructive bond between the film and the substrate. Non-limiting examples of suitable substrates include glass, stainless steel, Teflon™ and polyethylene- or silicone-impregnated paper. Following casting, the film is then dried. Drying of the slurry can be carried out at high temperature with the aid of a drying oven, a drying terminal, a vacuum drier, or any other suitable drying equipment known in the art. In other embodiments, the film is made by extrusion of the film composition through a die, followed by cutting to a desired thickness, and drying. In other embodiments, the film is made by solvent casting.

The films of the present invention may be made by formulation from a slurry, where the dry film comprises the following:
(a) about 5 to about 50% of the first polymer based on dry weight; and
(b) about 5 to about 50% of the second polymer, based on dry weight.

In various embodiments, the film comprises about 10 to about 40%, about 12 to about 35%, and about 15 to about 30% of each of the first and second polymers based on dry weight.

The film fragments may be incorporated in the base compositions of the present invention at a broad range of concentrations. In various embodiments, the carrier may comprise fragments at a level of about 0.005 to about 15%, about 0.41 to about 12%, about 0.05 to about 10%, about 0.01 to about 8% or about 0.05 to about 5% by weight of the composition.

In certain embodiments, the compositions of the present invention comprise a film according to the present invention, in a carrier. As referred to herein, a "carrier" is any material or composition in which a film can be embedded and suspended, and is suitable for administration or application to a human or animal subject as an oral care or personal care formulation, or suitable for administration to a household surface as a home care formulation. In various embodiments comprising a plurality oftilm fragments, such fragments may be embedded, suspended dispersed or otherwise distributed in the carrier. Selection of the carrier depends upon the desired use of the film, *i.e.,* in oral care, personal care or home care compositions.

In various embodiments, the carrier is a liquid, semi-solid or solid. A "liquid" can be a liquid of low or high viscosity, and includes a liquid having a flowrate that is imperceptible under ambient conditions. For example, a soap, such as a bar of hand soap, can be considered a liquid as defined herein. A liquid can be a thixotropic liquid. A "semi-solid" as used herein can be a gel, a colloid, or a gum. As used herein, semi-solids and liquids are fluids distinguished on the basis of viscosity: a semi-solid is a high viscosity fluid, while a liquid has lower viscosity. There is no definitive dividing line between these two types of fluids. A semi-solid can, in certain embodiments, have a viscosity as high as thousands of mPa·s. Carriers useful herein include liquids, pastes, ointments, gels, and foams, and can be transparent, translucent or opaque.

In certain embodiments, the compositions of the present invention are oral care compositions suitable for administration to the oral cavity. Such compositions include dentifrices (including mouthwashes and mouthrinses), dental gels, lozenges, beads, gums, oral strips, mints, liquid toothpastes, sprays, paint-on gels, lip balms, whitening strips, breath strips, oral chews, dental flosses and combinations thereof. An oral care composition disclosed herein can be used, for example, for cavity prevention, whitening, plaque prevention or reduction, gingivitis prevention or reduction, control of calculus, sensitivity prevention or reduction, or breath malodor prevention or reduction, and stain prevention.

In certain embodiments, a composition of the present invention can be a skin care composition, for example, a soap, a lotion, a body wash, a bath gel, a shampoo, a conditioner, a deodorant, an antiperspirant, a fragrance, a perfume, a cosmetic or combinations thereof, such as a antiperspirant/deodorant. In certain embodiments, a composition of the present invention can be a hair care composition, such as, for example, a shampoo or a conditioner, or a combination thereof.

In certain embodiments, a composition of the present invention can be a home care composition, for example, a dishwashing detergent, a laundry detergent, a fabric softener, a hard surface cleaner or a bleach composition. Useful carriers for home care compositions include, for example, surfactants, detergents and foaming agents.

The specific composition of the carrier preferably depends on the intended use of the composition. In various embodiments, the carrier is aqueous, comprising about 5 to about 95% water, about 10 to about 80% water or about 15 to about 75% water. In other embodiments, the carrier is substantially non-aqueous. In various embodiments, the carrier may be a dentifrice carrier having a water content of about 5% to about 70%, about 10% to about 50%, or about 20% to about 40%. In other embodiments, the non-aqueous dentifrice carrier comprises less than about 5% water.

The carrier may comprise any of a variety of materials, including emulsifiers, thickeners, fillers, and preservatives. In some embodiments, the carrier comprises a functional material, such as those described above. In some embodiments, the carrier comprises the same functional material as the film.

In certain embodiments, the carrier is suitable for use as a dentifrice. In some embodiments, the carrier comprises a humectant, such as glycerine, sorbitol or an alkylene glycol such as polyethylene glycol or propylene glycol. In some configurations, the carrier comprises a humectant at a level of about 10% to about 80% by weight, or about 20% to about 60% by weight of the composition. Carrier compositions among those useful herein are disclosed in. *e.g.,* U.S. Patents 5,695,746 to Garlick, Jr., et al, and 4,839.157 to Mei-King Ng et al.

In various dentifrice embodiments, the carrier comprises thickeners, gelling agents or combinations thereof. Thickeners or gelling agents useful herein include inorganic, natural or synthetic thickeners or gelling agents. In some configurations, the carrier comprises the thickener and gelling agent at total levels of about 0.10% to about 15% by weight, or about 0.4% to about 10% by weight of the composition. Examples of thickeners and gelling agents useful herein include inorganic thickening silicas such as: an amorphous silica, for example Zeodent® 165 (Huber Corporation); Irish moss; iota-carrageenan; gum tragacanth; or polyvinylpyrrolidone. In certain embodiments, the carrier comprises a polishing agent, such as a silica, a calcined alumina, sodium bicarbonate, calcium carbonate, dicalcium phosphate or calcium pyrophosphate. In various embodiments, the carrier can be a visually clear composition.

In certain dentifrice embodiments comprising a visually clear carrier, the composition comprises at least one polishing agent. Polishing agents among those useful herein include collodial silica, such as, for example, Zeodent® 115 (Huber Corporation), and alkali metal aluminosilicate complexes (*i.e.,* a silica comprising alumina). In some configurations, a polishing agent can have a refractive index close to that of a gelling agent combined with water and/or humectant. In various embodiments, the carrier comprises the polishing agent at a level of about 5% to about 70% by weight of the composition.

In certain dentifrice embodiments, the carrier comprises a surfactant or mixture of surfactants. Useful surfactants include water-soluble salts of at least one higher fatty acid monoglyceride monosulfate, such as the sodium salt of the monsulfated monoglyceride of hydrogenated coconut oil fatty acids: cocamidopropyl betaine: a higher alkyl sulfate such as sodium lauryl sulfate; an alkyl aryl sulfonate such as sodium dodecyl benzene sulfonate; a higher alkyl sulfoacetate; sodium lauryl sulfoacetate; a higher fatty acid ester of 1,2-dihydroxy propane sulfonate; and a substantially saturated higher aliphatic acyl amides of a lower aliphatic amino carboxylic acid, such as those having 12 to 16 carbons in the fatty acid, alkyl or acyl radicals; and mixtures thereof. Amides can be, for example, N-lauroyl sarcosine, and the sodium, potassium, and ethanolamine salts of N-lauroyl, N-myristoyl, or N-palmitoyl sarcosine. In various embodiments the carrier comprises the surfactant at a level of about 0.3 to about 15%, about 0.5% to about 10%, or about 1 to about 3% by weight of composition.

In certain embodiments, water-insoluble polymeric materials can be aqueous emulsions or dispersions of polymeric materials comprising polymers. In some configurations, the polymers can comprise precursor monomers, mixtures of monomers, natural polymers and mixtures thereof. In some configurations, a polymeric material can also include water-insoluble polymeric materials. In certain configurations, a water-insoluble polymer can comprise about 3% to about 60%; about 4% to about 40%, or about 5% to about 30% by weight of the composition. In non-limiting example, a water-insoluble polymeric material can comprise monomers chosen from aromatic vinyls, dienes, vinyl cyanides, vinyl halides, vinylidene halides, vinyl esters, olefins and their isomers, vinyl pyrrolidone, unsaturated carboxylic acids, alkyl esters of unsaturated carboxylic acids, hydroxy derivatives of alkyl esters of unsaturated carboxylic acids, amides of unsaturated carboxylic acids, amine derivatives of unsaturated carboxylic acids, glycidyl derivatives of alkyl esters of unsaturated carboxylic acids, olefinic diamines and isomers, aromatic diamines, terephthaloyl halides, olefinic polyols and mixtures thereof.

In various embodiments, a composition comprising a carrier and a film fragment in accordance with the present invention can be suitable for use as an antiperspirant, a deodorant, or an antiperspirant /deodorant, a shampoo, a lotion.

### Methods of Use:

Also disclosed herein are methods for the administering a film composition to a subject such as a human or animal. As referred to herein, "administering" refers to any method by which a composition is applied on or administered to the subject. In various embodiments, the administration is topical, wherein the composition is applied to an external surface of the subject, such as to the oral surfaces (*e.g*., teeth, gums and tongue), to the skin, to the eye, and to the hair. The specific route and method of administration will depend upon the intended use of the composition.

In various embodiments, the present invention provides methods for administering a functional material to a human or animal subject in need thereof, comprising topically applying to said subject a composition comprising a film in a carrier, wherein said film comprises the functional material. In one embodiment, the method additionally comprises disrupting the film after topically applying the film. Such disruption may be accomplished by any of a variety of methods, including chemical and/or mechanical means. Chemical means include degradation of the film by contact with water or a material present at the site of administration (*e.g*., saliva in an oral care application). Physical means include agitation, grinding, and shear forces produced by application of physical energy to the composition during use (*e.g*., brushing in a dentifrice application or scrubbing or wiping in a home care application).

In various embodiments, the present invention provides methods for the treatment of an oral care condition. As referred to herein, an "oral care condition" is any disorder or condition that can be prevented or treated by administration of a composition to the oral cavity, including disorders or conditions of the teeth, oral mucosa, gingiva and tongue. Such conditions include caries, gingivitis, periodontitis, sensitivity, dry mouth, buildup of calculus and cosmetic conditions such as staining, loss of tooth enamel or malodor.

In certain embodiments, the present invention provides methods for maintaining the systemic health of a mammal, comprising the steps of administering to the mammal a composition comprising a film according to the present invention.

The present invention can be further understood by reference to the following non-limiting Examples.

### EXAMPLES

Various slurries were prepared in accordance with Table 1, with varying proportions of methylcellulose (MC) and hydroxypropylmethylcellulose (HPMC), as follows:

**Table 1: Formulations Tested**

| **Ingredient** | **Slurry wt% (Example 1)** | **Slurry wt% (Example 2)** | **Slurry wt% (Example 3)** | **Slurry wt% (Example 4)** |
|---|---|---|---|---|
| Water | 72.5 | 79.3 | 80.5 | 81 |
| Methocel E5 (HPMC) | 10 | 0 | 3 | 6 |
| Methocel E50 (HPMC) | 3 | 0 | 0 | 0 |
| Methocel A15 (MC) | 0 | 9 | 6 | 3 |
| Corn starch | 4 | 2 | 3 | 3 |
| Flavorant | 7 | 7 | 6 | 6 |
| Pigmentation | 1 | 0.5 | 0.5 | 0.25 |
| Propylene Glycol | 2 | 1.5 | 0.5 | 0.25 |
| Surfactant | 0.5 | 0.5 | 0.5 | 0.5 |

The slurries were then cast into films, which were then tested to determine various physical characteristics. Stability of the films in a dentifrice carrier was evaluated after 1 week at controlled room temperature by: (1) visual determination that no change in shape or size occurred; and (2) tactile determination that shapes did not break apart with gentle rubbing. The specific dentifrice used in this evaluation was prepared using ingredients and methods known in the art, but specifically containing 2% organic acid polymer, e.g., polyvinylalkyl ether-maleic acid copolymer (such as, for example, Gantrez®). Results of the tests are shown below:

**Table 2: Properties of Films Tested (Examples 1 through 4, of which Examples 1 and 2 are not in accordance with the present invention)**

| **Film Formula** | **Breaking Strength (psi)** | **Stable? (Y/N)** | **Dissolution time in Water (sec)** |
|---|---|---|---|
| Example 1 (all HPMC) | >3000 psi (>20,682 kPa) | N | <120 |
| Example 2 (all MC) | very weak - not tested | Y | 577 |
| Example 3 (1:2 HPMC:MC) | 775 psi (5,343 kPa) | Y | 506 |
| Example 4 (2:1 HPMC:MC) | 1686 psi (11,623 kPa) | Y | 105 |

Results indicated that the film of Examples 3 and 4 have a higher breaking strength than the film of Example 2, while maintaining a shorter dissolution time.

## Claims

1. A film comprising:
(a) 5 to 50% of a cellulosic composition chosen from hydroxypropylmethylcellulose or hydroxypropylcellulose based on dry weight; and
(b) 5 to 50% of methyl cellulose based on dry weight;
wherein the breaking strength of the film is greater than 750 psi (5,171 kPa).

2. The film according to claim 1, comprising:
(a) 10 to 40% hydroxypropylmethylcellulose or hydroxypropylcellulose based on dry weight; and
(b) 10 to 40% of methyl cellulose based on dry weight.

3. The film according to any one of claims 1 to 2, wherein the film has a substantially lamellar structure.

4. The film according to any one of claims 1 to 3, wherein the cellulosic composition of (a) is hydroxypropylmethylcellulose.

5. A composition comprising the film of any one of claims 1 to 4 in a carrier.

6. The composition according to claim 5, further comprising a functional material selected from: an abrasive, an anticaries agent, an anticalculus agent, an antiplaque agent, a periodontal active, a breath freshening agent, a malodor control agent, a whitening agent, a stain prevention active, a salivary stimulant, an amino acid, and a combination of two or more thereof.

7. The composition according to claim 5 or claim 6, in the form of an oral care composition.

8. The composition according to claim 5, wherein the composition is in the form of a soap, a lotion, a body wash, a bath gel, a shampoo, a conditioner, a deodorant, an antiperspirant, a fragrance, a perfume or a cosmetic.

9. The composition according to claim 5, further comprising an active agent chosen from:
foaming agents, surfactants, cleansing agents, bleaches, detergents, or fabric softening agents.

## Patentansprüche

1. Film, der Folgendes umfasst:
(a) 5 bis 50 % einer cellulosehaltigen Zusammensetzung, die aus Hydroxypropylmethylcellulose oder Hydroxypropylcellulose ausgewählt ist, bezogen auf das Trockengewicht; und
(b) 5 bis 50 % Methylcellulose, bezogen auf das Trockengewicht;
wobei die Bruchfestigkeit des Films größer als 750 psi (5 171 kPa) ist.

2. Film nach Anspruch 1, der Folgendes umfasst:
(a) 10 bis 40 % Hydroxypropylmethylcellulose oder Hydroxypropylcellulose, bezogen auf das Trockengewicht; und
(b) 10 bis 40 % Methylcellulose, bezogen auf das Trockengewicht.

3. Film nach einem der Ansprüche 1 bis 2, wobei der Film eine im Wesentlichen lamellare Struktur aufweist.

4. Film nach einem der Ansprüche 1 bis 3, wobei die cellulosehaltige Zusammensetzung von (a) Hydroxypropylmethylcellulose ist.

5. Zusammensetzung, die den Film nach einem der Ansprüche 1 bis 4 in einem Träger umfasst.

6. Zusammensetzung nach Anspruch 5, die weiter einen funktionellen Stoff umfasst, der aus Folgenden ausgewählt ist: einem Schleifmittel, einem Antikariesmittel, einem Antizahnsteinmittel, einem Antiplaquemittel, einem parodontalen Wirkstoff, einem Atemfrischemittel, einem Geruchskontrollmittel, einem Weißmacher, einemWirkstoff zur Fleckenverhinderung, einem Speichelstimulans, einer Aminosäure und einer Kombination von zwei oder mehr davon.

7. Zusammensetzung nach Anspruch 5 oder Anspruch 6 in der Form einer Mundpflegezusammensetzung.

8. Zusammensetzung nach Anspruch 5, wobei die Zusammensetzung in der Form von Folgenden vorliegt: einer Seife, einer Lotion, eines Duschgels, eines Badegels, eines Shampoos, einer Haarspülung, eines Deodorants, eines Antitranspirants, eines Duftstoffs, eines Parfüms oder eines Kosmetikums.

9. Zusammensetzung nach Anspruch 5, die weiter einen Wirkstoff umfasst, der aus Folgenden ausgewählt ist: Schaummitteln, Tensiden, Reinigungsmitteln, Bleichmitteln, Waschmitteln oder Weichspülern.

## Revendications

1. Un film comprenant :
(a) 5 à 50% d'une composition cellulosique choisie parmi l'hydroxypropylméthylcellulose ou l'hydroxypropylcellulose sur la base du poids sec ; et
(b) 5 à 50% de méthyl cellulose sur la base du poids sec ;
dans lequel la résistance à la rupture du film est supérieure à 750 psi (5171 kPa).

2. Le film selon la revendication 1, comprenant :
(a) 10 à 40% d'hydroxypropylméthylcellulose ou d'hydroxypropylcellulose sur la base du poids sec ; et
(b) 10 à 40% de méthyl cellulose sur la base du poids sec.

3. Le film selon l'une quelconque des revendications 1 ou 2, dans lequel le film a une structure sensiblement lamellaire.

4. Le film selon l'une quelconque des revendications 1 à 3, dans lequel la composition cellulosique de (a) est l'hydroxypropylméthylcellulose.

5. Une composition comprenant le film selon l'une quelconque des revendications 1 à 4 dans un excipient.

6. La composition selon la revendication 5, comprenant en outre un matériau fonctionnel sélectionné parmi : un abrasif, un agent anti-carie, un agent antitartre, un agent anti-plaque, un actif parodontal, un agent de rafraîchissement de l'haleine, un agent de lutte contre les mauvaises odeurs, un agent de blanchiment, un actif de prévention des taches, un stimulant salivaire, un acide aminé et une combinaison d'au moins deux de ceux-ci.

7. La composition selon la revendication 5 ou la revendication 6, sous la forme d'une composition de soins buccaux.

8. La composition selon la revendication 5, dans laquelle la composition est sous la forme d'un savon, d'une lotion, d'un gel corporel, d'un gel de bain, d'un shampooing, d'un après-shampooing, d'un déodorant, d'un anti-transpirant, d'une fragrance, d'un parfum ou d'un produit de beauté.

9. La composition selon la revendication 5, comprenant en outre un agent actif choisi parmi :
les agents moussants, les tensioactifs, les agents de nettoyage, l'eau de Javel, les détergents ou les agents assouplissants.
